# EUROPEAN PATENT SPECIFICATION

(11) **EP 2 013 349 B1**
(45) Date of publication and mention of the grant of the patent: **17.11.2010**
(21) Application number: 07728440.4
(22) Date of filing: 24.04.2007
(51) Int. Cl.: C12N 15/67

(54) **SELECTION OF HOST CELLS EXPRESSING PROTEIN AT HIGH LEVELS**
SELEKTION VON WIRTSZELLEN MIT PROTEINEXPRESSION AUF HOHEM NIVEAU
SÉLECTION DE CELLULES HÔTES EXPRIMANT UNE PROTÉINE À DE HAUTS NIVEAUX

(30) Priority: 02.05.2006 US 416490; 02.05.2006 EP 06113354
(43) Date of publication of application: 14.01.2009
(73) Proprietor: ChromaGenics B.V., 2333 CN Leiden (NL)
(72) Inventor: OTTE, Arie, Pieter, 3823 SG Amersfoort (NL); VAN BLOKLAND, Henricus, Johannes, Maria, 1456 NH Wijdewormer (NL); KWAKS, Theodorus, Hendrikus, Jacobus, 1022 XW Amsterdam (NL); SEWALT, Richard, George, Antonius, Bernardus, 6814 HD Arnhem (NL)
(74) Representative: Manten, Annemieke
(86) International application number: PCT/EP2007/053984
(87) International publication number: WO 2007/128685

(56) References cited:
- WO-A-96/12008
- US-A1- 2006 195 935
- RAZIN AHARON: "CpG methylation, chromatin structure and gene silencing - a three-way connection" EMBO JOURNAL, OXFORD UNIVERSITY PRESS, SURREY, GB, vol. 17, no. 17, 1998, pages 4905-4908, XP002233552 ISSN: 0261-4189
- BIRD ADRIAN P ET AL: "Methylation-induced repression: Belts, braces, and chromatin" CELL, vol. 99, no. 5, 24 November 1999 (1999-11-24), pages 451-454, XP002446533 ISSN: 0092-8674
- WILLIAMS STEVEN ET AL: "CpG-island fragments from the HNRPA2B1/CBX3 genomic locus reduce silencing and enhance transgene expression from the hCMV promoter/enhancer in mammalian cells" BMC BIOTECHNOLOGY, BIOMED CENTRAL LTD. LONDON, GB, vol. 5, no. 1, 3 June 2005 (2005-06-03), page 17, XP021005966 ISSN: 1472-6750
- VAN BLOKLAND ET AL: "A novel, high stringency selection system allows screening of few clones for high protein expression" JOURNAL OF BIOTECHNOLOGY, ELSEVIER SCIENCE PUBLISHERS, AMSTERDAM, NL, vol. 128, no. 2, 13 January 2007 (2007-01-13), pages 237-245, XP005829332 ISSN: 0168-1656
- KWAKS T H J ET AL: "Targeting of a histone acetyltransferase domain to a promoter enhances protein expression levels in mammalian cells" JOURNAL OF BIOTECHNOLOGY, ELSEVIER SCIENCE PUBLISHERS, AMSTERDAM, NL, vol. 115, no. 1, 12 January 2005 (2005-01-12), pages 35-46, XP004966987 ISSN: 0168-1656
- KWAKS ET AL: "Employing epigenetics to augment the expression of therapeutic proteins in mammalian cells" TRENDS IN BIOTECHNOLOGY, ELSEVIER PUBLICATIONS, CAMBRIDGE, GB, vol. 24, no. 3, March 2006 (2006-03), pages 137-142, XP005318530 ISSN: 0167-7799
- OTTE ARIE P ET AL: "Various Expression-Augmenting DNA Elements Benefit from STAR-Select, a Novel High Stringency Selection System for Protein Expression." BIOTECHNOLOGY PROGRESS 2007 JUL-AUG, vol. 23, no. 4, July 2007 (2007-07), pages 801-807, XP002446534 ISSN: 8756-7938

## Description

### BACKGROUND OF THE INVENTION

Field of the Invention: The invention relates to the field of molecular biology and biotechnology. More specifically the present invention relates to means and methods for improving the selection of host cells that express proteins at high levels.

Proteins can be produced in various host cells for a wide range of applications in biology and biotechnology, for instance as biopharmaceuticals. Eukaryotic and particularly mammalian host cells are preferred for this purpose for expression of many proteins, for instance when such proteins have certain posttranslational modifications such as glycosylation. Methods for such production are well established, and generally entail the expression in a host cell of a nucleic acid (also referred to as 'transgene') encoding the protein of interest. In general, the transgcnc together with a selectable marker gene is introduced into a precursor cell, cells are selected for the expression of the selectable marker gene, and one or more clones that express the protein of interest at high levels are identified, and used for the expression of the proteins of interest.

Methods to select recombinant host cells expressing relatively high levels of desired proteins are known (see, *e.g*. introductions in WO 2006/048459 and US 2006/0141577).

A novel concept for selecting host cells expressing high levels of polypeptides of interest was disclosed in international application PCT/EP2005/055794 (published as WO 2006/048459), which was filed before but published after the priority date of the instant application. An alternative was disclosed in US patent application no. 11/359,953 (published as US 2006/0141577) and in international application PCT/EP2007/051696, also filed before but published after the priority date of the instant application. Briefly, those applications teach the use of a sequence encoding a selectable marker polypeptide with a non-ATG startcodon, e.g. a GTG or TTG. This resulted in the possibility to select clones with high stringency and was used to obtain clones of host cells with very high expression levels.

The present invention aims at providing further improved means and methods for selection of host cells expressing high levels of proteins of interest.

### BRIEF SUMMARY OF THE INVENTION

PCT/EP2005/055794 (WO 2006/048459), US 11/359,953 (US 2006/0141577) and PCT/EP2007/051696 teach the use of a sequence encoding a selectable marker polypeptide with a non-ATG startcodon, e.g. a GTG or TTG. This resulted in the possibility to select clones with high stringency and was used to obtain clones of host cells with very high expression levels.

The present invention discloses improved selectable marker genes with a GTG or TTG startcodon. Such improved selectable marker genes can for instance be used in the transcription units and methods of use thereof described in WO 2006/048459 and US 2006/0141577. This leads to further improved (selection of) host cells with high expression levels.

In one aspect, the invention provides a DNA molecule comprising an open reading frame sequence that encodes a selectable marker polypeptide, wherein said DNA molecule in the coding stand comprises a translation start sequence for the selectable marker polypeptide chosen from the group consisting of: a) a GTG startcodon: and b) a TTG startcodon; and wherein the open reading frame sequence that encodes the selectable marker protein has been mutated to replace at least half of its CpG dinucleotides as compared to the native open reading frame sequence that encodes the selectable marker protein.

According to the invention, the selectable marker protein provides resistance against lethal and/or growth-inhibitory effects of a selection agent, such as an antibiotic. In particular, the selectable marker polypeptide provides resistance against zeocin or against neomycin.

The invention further provides a DNA molecule according to the invention, wherein the open reading frame sequence that encodes a selectable marker polypeptide is part of a multicistronic transcription unit that further comprises an open reading frame sequence encoding a polypeptide of interest.

The invention further provides an expression cassette comprising such DNA molecules, said expression cassette comprising a promoter upstream of said multicistronic transcription unit and preferably a transcription termination sequence downstream of the multicistronic transcription unit.

The invention further provides host cells comprising a DNA molecule or an expression cassette according to the invention.

The invention further provides a method of expressing a polypeptide of interest, comprising culturing a host cell comprising the expression cassette of the invention, and expressing the polypeptide of interest from the expression cassette.

### LEGENDS TO THE FIGURES

FIG. 1. Results with a zeocin resistance marker with reduced CpG content in CHO-K1 cells. Dots indicate individual data points; lines indicate the average expression levels; vertical axis indicates d2EGFP signal. See example 1 for details.

FIG. 2. As Fig. 1, but now in CHO-DG44 cells. See example 1 for details.

FIG. 3. Results with 'CpG poor' neomycin resistance marker having different mutations. Dots indicate individual data points; lines indicate the average expression levels; vertical axis indicates d2EGFP signal. See example 2 for details.

### DETAILED DESCRIPTION OF THE INVENTION

The term "monocistronic gene" is defined as a gene capable of providing a RNA molecule that encodes one polypeptide. A "multicistronic transcription unit", also referred to as multicistronic gene, is defined as a gene capable of providing an RNA molecule that encodes at least two polypeptides. The term "bicistronic gene" is defined as a gene capable of providing a RNA molecule that encodes two polypeptides. A bicistronic gene is therefore encompassed within the definition of a multicistronic gene. A "polypeptide" as used herein comprises at least five amino acids linked by peptide bonds, and can for instance be a protein or a part, such as a subunit, thereof. It may comprise posttranslational modifications, e.g. glycosylation. Mostly, the terms polypeptide and protein are used interchangeably herein. A "gene" or a "transcription unit" as used in the present invention can comprise chromosomal DNA, cDNA, artificial DNA, combinations thereof, and the like. "Operably linked" refers to a situation where the components described are in a relationship permitting them to function in their intended manner. Thus, for example, a promoter "operably linked" to a cistron is ligated in such a manner that expression of the cistron is achieved under conditions compatible with the promoter. Similarly, a nucleotide sequence of an IRES operably linked to a cistron is ligated in such a manner that translation of the cistron is achieved under conditions compatible with the IRES.

The DNA molecules of the invention can be present in the form of double stranded DNA, having with respect to the selectable marker polypeptide and the polypeptide of interest a coding strand and a non-coding strand, the coding strand being the strand with the same sequence as the translated RNA, except for the presence of T instead of U. Hence, an AUG startcodon is coded for in the coding strand by an ATG sequence, and the strand containing this ATG sequence corresponding to the AUG startcodon in the RNA is referred to as the coding strand of the DNA. It will be clear to the skilled person that startcodons or translation initiation sequences are in fact present in an RNA molecule, but that these can be considered equally embodied in a DNA molecule coding for such an RNA molecule; hence, wherever the present invention refers to a startcodon or translation initiation sequence, the corresponding DNA molecule having the same sequence as the RNA sequence but for the presence of a T instead of a U in the coding strand of said DNA molecule is meant to be included, and vice versa, except where explicitly specified otherwise. In other words, a startcodon is for instance an AUG sequence in RNA but the corresponding ATG sequence in the coding strand of the DNA is referred to as startcodon as well in the present invention. The same is used for the reference of 'in frame' coding sequences, meaning triplets (3 bases) in the RNA molecule that are translated into an amino acid, but also to be interpreted as the corresponding trinucleotide sequences in the coding strand of the DNA molecule.

A translation start sequence is often referred to in the field as 'Kozak sequence', and an optimal Kozak sequence is RCCATGG, the startcodon underlined, R being a purine, i.e. A or G (see Kozak M, 1986, 1987, 1989, 1990, 1997, 2002). Hence, besides the startcodon itself, the context thereof, in particular nucleotides --3 to -1 and +4, are relevant, and an optimal translation startsequence comprises an optimal startcodon (i.e. ATG) in an optimal context (i.e. the ATG directly preceded by RCC and directly followed by G). Translation by the ribosomes is most efficient when an optimal Kozak sequence is present (see Kozak M, 1986, 1987, 1989, 1990, 1997, 2002). However, in a small percentage of events, non-optimal translation initiation sequences are recognized and used by the ribosome to start translation. The present invention makes use of this principle, and allows for decreasing the amount of translation and hence expression of the selectable marker polypeptide, which can therefore be used to increase the stringency of the selection system.

The term "selection marker" or "selectable marker" is typically used to refer to a gene and/or protein whose presence can be detected directly or indirectly in a cell, for example a polypeptide that inactivates a selection agent and protects the host cell from the agent's lethal or growth-inhibitory effects (e.g. an antibiotic resistance gene and/or protein). Selectable marker polypeptides are well known in the art and routinely used when eukaryotic host cell clones are to be obtained, and several examples of suitable selectable marker proteins are provided in WO 2006/048459, DNA sequences coding for such selectable marker polypeptides are known, and several examples of wild-type sequences of DNA encoding selectable marker proteins are provided in WO 2006/048459 (e.g. Figs. 15-21 therein). It will be clear that mutants or derivatives of selectable markers can also be suitably used, and are therefore included within the scope of the term 'selectable marker polypeptide' as long as the selectable marker protein is still functional. For instance any silent mutations that do not alter the encoded protein because of the redundancy of the genetic code are also encompassed. Further mutations that lead to conservative amino acid mutations or to other mutations are also encompassed, as long as the encoded protein still has activity, which may or may not be lower than that of the wild-type protein as encoded by the indicated sequences. In particular, it is preferred that the encoded protein is at least 70%, preferably at least 80%, more preferably at least 90%, still more preferably at least 95% identical to the proteins encoded by the respective indicated sequences. Testing for activity of the selectable marker proteins can be done by routine methods. A selectable marker polypeptide according to the invention is a protein that is encoded by nucleic acid, which polypeptide can be functionally used for selection, for instance because it provides resistance to a selection agent such as an antibiotic. Hence, when an antibiotic is used as a selection agent, the DNA encodes a polypeptide that confers resistance to the selection agent, which polypeptide is the selectable marker polypeptide. The selectable marker polypeptide is encoded by the DNA of the invention. The selectable marker polypeptide according to the invention must be functional in a eukaryotic host cell, and hence being capable of being selected for in eukaryotic host cells. Examples of suitable selectable marker genes for the present invention are zeocin and neomycin. Other suitable candidates include e.g. blasticidin, puromycin, bleomyoin, hygromycin. DHFR, GS, etc (see also WO 2006/048459). Other selectable marker genes that could be used, and their selection agents, are for instance described in table 1 of US patent 5,361,053: see also Kaufman. Methods in Enzymology, 185:537-566 (1990), for a review of these. The term "selection" is typically defined as the process of using a selection marker/selectable marker and a selection agent to identify host cells with specific genetic properties (e.g. that the host cell contains a transgene integrated into its genome), For convenience and as generally accepted by the skilled persons, in many publications as well as herein, often the gene and protein encoding the resistance to a selection agent is referred to as the 'selectable agent (resistance) gene' or 'selection agent (resistance) protein', respectively, although the official names may be different, e.g. the gene coding for the protein conferring resistance to neomycin (as well as to G418 and kanamycin) is often referred to as neomycin (resistance) (or neo^{r}) gene, while the official name is aminoglycoside 3'-phosphotransferase gene.

The coding sequences of the selectable marker protein of WO 2006/048459 and US 2006/0141577 in preferred embodiments have a GTG or more preferably a TTG startcodon. This results in very stringent selection and very high expression of the protein of interest in the clones that are obtained. In the present invention, the coding sequences of the selectable marker protein are further improved by reducing the CpG content therein, resulting in even higher stringency and further improved expression levels.

Preferably, the translation start sequence in the coding strand for the selectable marker polypeptide comprises a TTG startcodon. Preferably, the GTG or TTG startcodon is flanked by sequences providing for relatively good recognition of the non-ATG sequences as startcodons, such that at least some ribosomes start translation from these startcodons, i.e. the translation start sequence preferably comprises the sequence ACC[GTG or TTG startcodon]G or GCC[GTG or TTG startcodon]G.

In one aspect, the invention provides a DNA molecule comprising an open reading frame sequence that encodes a selectable marker polypeptide, wherein said DNA molecule in the coding strand comprises a translation start sequence for the selectable marker polypeptide chosen from the group consisting of: a) a GTG startcodon; and b) a TTG startcodon; and wherein the open reading frame sequence that encodes the selectable marker protein has been mutated to replace at least 10% of its CpG dinucleotides (any 'CG' in the sequence) as compared to the native open reading frame sequence that encodes the selectable marker protein. Such a DNA molecule can be used according to the invention for obtaining eukaryotic host cells expressing high levels of the polypeptide of interest, by selecting for the expression of the selectable marker polypeptide. Subsequently or simultaneously, one or more host cell(s) expressing the polypeptide of interest can be identified, and further used for expression of high levels of the polypeptide of interest.

It is shown herein that the reduction of the CpG content of the selectable marker gene of the invention, i.e. having a TTG or GTG startcodon, can lead to improved expression of a polypeptide of interest that is translated from a multicistronic transcription unit from which also the selectable marker polypeptide is translated. Without wishing to be bound by theory, it is believed that reduction of the CpG content may reduce the possibility for silencing of transcription, because CpG dinucleotides can be methylated and silenced in eukaryotes. Selectable marker polypeptides that are encoded by genes with a relatively high CpG content, often derived from bacterial sequences, for instance zeocin and neomycin, may benefit most from the reduction of the CpG content, although some benefit may already be found for selection genes with a relatively low CpG content. In certain embodiments, CpG dinucleotides are removed from a sequence encoding a selectable marker polypeptide without changing the encoded amino acid sequence. This can be done by taking advantage of the redundancy of the genetic code, as is well known and routine to the person skilled in the art of molecular biology.

It is expected that a positive effect of removing CpG dinucleotides will be apparent when at least 10% of the CpG dinucleotides in the coding sequence of the selectable marker gene have been replaced. It is expected that removal of more CpG dinucleotides will increase the effect, and hence in certain embodiments, at least 20%, at least 30%, at least 40%, at least 50%, at least 60%, at least 70% or at least 80% of the CpG dinucleotides are mutated compared to the native open reading frame sequence that encodes the selectable marker protein. In certain advantageous embodiments, at least half of the CpG dinucleotides of the open reading frame sequence that encodes the selectable marker polypeptide have been replaced as compared to the native open reading frame sequence that encodes the selectable marker polypeptide.

A native open reading frame sequence that encodes the selectable marker polypeptide that provides resistance to zeocin is given as SEQ. ID. NO. 1 (containing internal ATGs), and mutation of A at position 280 into T in this sequence gives a sequence lacking internal ATGs, and wherein the internally encoded methionine at position 94 is replaced by leucine. For the DNA sequences of the invention, the startcodon (first three nucleotides of the DNA sequences) is mutated into a GTG or into a TTG startcodon.

In certain advantageous embodiments, the selectable marker polypeptide provides resistance against zeocin. In certain embodiments thereof, the DNA molecule comprises SEQ, ID. NO. 1, wherein at least half of the CpG dinucleotides has been replaced without mutating the amino acid sequence that is encoded, with the proviso that the startcodon (first three nucleotides in the sequence) is replaced by a startcodon chosen from GTG or TTG. In an alternative embodiment, the DNA molecule comprises SEQ. ID. NO. 1 wherein nucleotide A at position 280 is replaced by T. such that encoded amino acid 94 (methionine) is replaced by leucine, and wherein at least half of the CpG dinucleotides has been replaced without further mutating the amino acid sequence that is encoded, with the proviso that the startcodon (first three nucleotides in the sequence) is replaced by a startcodon chosen from GTG or TTG. This embodiment tacks ATG sequences in the coding sequence for the zeocin resistance gene, and is therefore suitable in the multicistronic transcription units of the invention wherein the coding sequence for the selectable marker polypeptide is upstream of the coding sequence for the polypeptide of interest. In one preferred embodiment hereof, the DNA molecule comprises SEQ. ID. NO. 3.

A native open reading frame sequence that encodes the selectable marker polypeptide that provides resistance to neomycin is given as SEQ. ID. NO. 5 (containing internal ATGs) and as SEQ. ID. NO. 7 (lacking internal ATGs). In advantageous embodiments, these sequences may contain one or more further mutations so that the encoded polypeptide has a mutation of valine at position 201 to glycine (201V>G), of glutamic acid at position 185 to aspartic acid (185E>D), or both (185E>D, 201V>G).

In other advantageous embodiments, the selectable marker polypeptide provides resistance against neomycin. In certain embodiments thereof, the DNA molecule comprises a sequence chosen from the group consisting of any one of: a) SEQ. ID. NO. 5, with the proviso that at least half of the CpG dinucleotides has been replaced without mutating the amino acid sequence that is encoded, and with the further proviso that the startcodon (the first ATG sequence) is replaced by either GTG or TTG; b) SEQ. ID. NO. 7. with the proviso that at least half of the CpG dinucleotides has been replaced without mutating the amino acid sequence that is encoded, and with the further proviso that the startcodon (the first ATG sequence) is replaced by either GTG or TTG; and c) SEQ. ID. NO. 5 or SEQ. ID. NO. 7, containing a mutation to encode a neomycin resistance protein variant as compared to the sequences encoded by the indicated sequences, said variant having glycine at position 201 in the encoded protein (201 G variant), or aspartic acid at position 185 (185D variant), or both glycine at position 201 and aspartic acid at position 185 (1.85D, 201G variant), with the proviso that at least half of the CpG dinucleotides in the given DNA sequence has been replaced without further mutating the amino acid sequence that is encoded, and with the further proviso that the startcodon (the first ATG sequence) is replaced by either GTG or TTG. The 185D variant is for instance obtained by replacing the codon from position 553-555 in the provided nucleic acid sequences with the sequence GAC, and the 201G variant is for instance obtained by replacing the codon from position 601-603 in the provided nucleic acid sequence with GGT. In one preferred embodiment, the DNA molecule comprises SEQ. ID. NO. 9, with the proviso that nucleotide A at position 555 is replaced by C (to encode the 185E>D variant), and that nucleotide T at position 602 is replaced by G and that nucleotide G at position 603 is replaced by T (to encode the 201 V>G variant), and with the further proviso that the startcodon (ATG at positions 1-3) is replaced by either GTG or TTG. It will be clear to the skilled person that further variations can be prepared by the skilled person without departing from the teaching of the present invention, and such further variations are encompassed with the present invention as long as the startcodon is not ATG and the encoded protein provides resistance against neomycin (or G418). The 185D and 201G variants further improve the selection stringency according to the present invention.

In certain embodiments, the selectable marker polypeptide further comprises a mutation that reduces the activity of the selectable marker polypeptide compared to its wild-type counterpart. This may be used to increase the stringency of selection even further. As non-limiting examples, proline at position 9 in the zeocin resistance polypeptide may be mutated, e.g. to Thr or Phe, and for the neomycin resistance polypeptide, amino acid residue 182 or 261 or both may further be mutated (see e.g. WO 01/32901).

In principle, the DNA molecules of the invention, encoding the selectable marker polypeptide, may be used in any expression vector, e.g. as a monocistronic gene. They provide stringent selection criteria. In preferred embodiments however, the ORF that encodes a selectable marker polypeptide is part of a multicistronic transcription unit that further comprises an ORF sequence encoding a polypeptide of interest.

A multicistronic transcription unit according to the invention can for instance be a multicistronic transcription unit comprising sequences coding from 5' to 3' for a selectable market polypeptide and for a polypeptide of interest, or for instance a multicistronic transcription unit comprising sequences coding from 5' to 3' for a polypeptide of interest and for a selectable marker polypeptide. In the former case, the coding sequence for the selectable marker polypeptide is preferably devoid of ATG sequences in the coding strand (see WO 2006/048459). In the latter case, the polypeptide of interest is encoded upstream from the coding sequence for the selectable marker polypeptide and an internal ribosome entry site (IRES) is operably linked to the sequence encoding the selectable marker polypeptide, and hence the selectable marker polypeptide is dependent from the IRES for its translation (see US 2006/0141577). In one embodiment therefore, a multicistronic transcription unit of the invention comprises in the following order: a) a promoter; b) the sequence encoding the selectable marker protein; and c) a sequence encoding a protein of interest. In another embodiment, a multicistronic transcription unit of the invention comprises in the following order a) a promoter; b) a sequence encoding a protein of interest; and c) an internal ribosome entry site (IRES), operably linked to d) the sequence encoding the selectable marker protein.

In certain embodiments, the multicistronic transcription units comprise a third cistron downstream of the second cistron, said third cistron preferably operably linked to an IRES, and for instance encoding a second selectable marker polypeptide. This second selectable marker polypeptide in certain embodiments is DHFR; preferably with a GTG or TTG startcodon to allow for continuous selection in dhfr-deficient cells (see, e.g. PCT/EP2007/051696).

In certain embodiments, the invention provides an expression cassette comprising a DNA molecule of the invention, said expression cassette comprising a promoter upstream of a multicistronic transcription unit of the invention and a transcription termination sequence downstream thereof Said expression cassette is functional in a eukaryotic host cell for driving transcription of the multicistronic transcription unit

An 'expression cassette' as used herein is a nucleic acid sequence comprising at least a promoter functionally linked to a sequence of which expression is desired. Preferably, an expression cassette further contains transcription termination and polyadenylation sequences. Examples of suitable promoters and transcription termination/polyadenylation sequences are well known and readily available to the skilled person, and are for instance discussed in WO 2006/048459, p. 28-29.

Other regulatory sequences such as enhancers may also be included. The promoter must be capable of functioning in a eukaryotic host cell, i.e. it must be capable of driving transcription of the transcription unit. The promoter is thus operably linked to the transcription unit. The expression cassette may optionally further contain other elements known in the art, e.g. splice sites, to comprise introns, and the like. In the embodiments where the selectable marker polypeptide is encoded downstream of the polypeptide of interest, an IRES is operably linked to the cistron that contains the selectable marker polypeptide coding sequence. In the embodiments where the selectable marker polypeptide is encoded upstream of the polypeptide of interest, the sequence encoding the selectable marker polypeptide is devoid of ATG sequences in the coding strand.

As used herein, an "internal ribosome entry site" or "IRES" refers to an element that promotes direct internal ribosome entry to the initiation codon, such as normally an ATG, but in this invention preferably GTG or TTG, of a cistron (a protein encoding region), thereby leading to the cap-independent translation of the gene. IRES sequences and use thereof for expression are well known to the person skilled in the art, as taught in US 2006/0141577 and PCT/EP2007/051696. See also, e. g., Jackson R J, Howell M T, Kaminski A (1990) Trends Biochem Sci 15 (12): 477-83), Jackson R J and Kaminski, A. (1995) RNA 1 (10): 985-1000, Martinez-Salas, 1999, Venkatesan & Dasgupta, 2001, Rees et al, 1996, and Mizuguchi et al., 2000. An example of a suitable IRES sequence is given in example 19 of US 2006/0141577 (SEQ ID NO. 127 therein).

DNA molecules according to the invention can be generated by standard molecular biology methods available to the skilled person. For instance, native sequences, e.g. from commercially available plasmids, may be mutated by routine methods. Moreover, it is at present also possible to synthesise at will (if required using subcloning steps) DNA sequences that have sufficient length for an ORF of a selectable marker polypeptide, and such synthetic DNA sequences can nowadays be ordered commercially from various companies. Hence, using the teachings of the present invention, the person skilled in the art may design appropriate sequences according to the invention encoding a selectable marker polypeptide (with a GTG or TTG startcodon, and with reduced CpG content, and in certain embodiments having no internal ATGs), have this sequence synthesized, and test the DNA molecule for functionality of the encoded selectable marker by introducing the DNA molecule in eukaryotic host cells and test for expression of functional selectable marker polypeptide. The commercial availability of such sequences also makes feasible to provide without undue burden for selection marker coding sequences lacking internal ATG sequences, where the wild-type coding sequence of the selection marker polypeptide comprises several such internal ATGs (see WO 2006/048459).

In certain embodiments, a DNA molecule according to the invention is part of a vector, e.g. a plasmid. Such vectors can easily be manipulated by methods well known to the person skilled in the art, and can for instance be designed for being capable of replication in prokaryotic and/or eukaryotic cells. In addition, many vectors can directly or in the form of isolated desired fragment therefrom be used for transformation of eukaryotic cells and will integrate in whole or in part into the genome of such cells, resulting in stable host cells comprising the desired nucleic acid in their genome.

The vector used can be any vector that is suitable for cloning DNA and that can be used for transcription of nucleic acid of interest. When host cells are used it is preferred that the vector is an integrating vector. Alternatively, the vector may be an episomally replicating vector.

It is widely appreciated that chromatin structure and other epigenetic control mechanisms may influence the expression of transgenes in eukaryotic cells (e.g. Whitelaw et al 2001). The multicistronic expression units according to the invention form part of a selection system with a rather rigorous selection regime. This generally requires high transcription levels in the host cells of choice. To increase the chance of finding clones of host cells that survive the rigorous selection regime, and possibly to increase the stability of expression in obtained clones, it will generally be preferable to increase the predictability of transcription. Therefore, in preferred embodiments, an expression cassette according to the invention further comprises at least one chromatin control element. A chromatin control element' as used herein is a collective term for DNA sequences that may somehow have an effect on the chromatin structure and therewith on the expression level and/or stability of expression of transgenes in their vicinity (they function 'in cis', and hence are placed preferably within 5 kb, more preferably within 2 kb, still more preferably within 1 kb from the transgene) within eukaryotic cells. Such a chromatin control element preferably is chosen from the group consisting of an insulator sequence, a ubiquitous chromatin opener element (UCOE), matrix or scaffold attachment regions (MAR/SAR) and anti-repressor (STAR) sequences. Examples of chromatin control elements, as well as methods for obtaining and using them and functionally testing them, are given in WO 2006/048459, pages 32-37. In certain embodiments, said at least one chromatin control element is an anti-repressor element chosen from the group consisting of any one of SEQ. ID. NO. 1 through SEQ. ID. NO. 66 of WO 2006/048459, and fragments thereof. In certain embodiments thereof, said expression cassette comprises SEQ. ID. NO. 66 of WO 2006/048459, or a fragment thereof positioned upstream of the promoter that drives transcription of the multicistronic transcription unit. In other embodiments, the multicistronic transcription unit is flanked on both sides by at least one anti-repressor sequence chosen from the group consisting of any one of SEQ. ID. NO. 1 through SEQ. ID. NO. 65 of WO 2006/048459, or fragments thereof. Preferably, the chromatin control element is chosen from the group consisting of STAR67, STAR7, STAR9, STAR17, STAR27, STAR29, STAR43, STAR44, STAR45, STAR47, STAR61, or a functional fragment or derivative of said STAR sequences (see e.g. WO 2006/048459 for the sequences and preferred uses of these STAR elements).

A polypeptide of interest according to the invention can be any protein, and may be a monomeric protein or a (part of a) multimeric protein. A multimeric protein comprises at least two polypeptide chains. Non-limiting examples of a protein of interest according to the invention are enzymes, hormones, immunoglobulin chains, therapeutic proteins like anti-cancer proteins, blood coagulation proteins such as Factor VIII, multi-functional proteins, such as erythropoietin, diagnostic proteins, or proteins or fragments thereof useful for vaccination purposes, all known to the person skilled in the art.

The polypeptide of interest may be from any source, and in certain embodiments is a mammalian protein, an artificial protein (e.g. a fusion protein or mutated protein), and preferably is a human protein.

DNA molecules comprising multicistronic transcription units and/or expression cassettes according to the invention can be used for improving expression of nucleic acid, preferably in host cells. The terms "cell"/"host cell" and "cell line"/"host cell line" are respectively typically defined as a cell and homogeneous populations thereof that can be maintained in cell culture by methods known in the art, and that have the ability to express heterologous or homologous proteins. The invention further provides host cells comprising a DNA molecule or an expression cassette according to the present invention.

Prokaryotic host cells can be used to propagate and/or perform genetic engineering with the DNA molecules of the invention, especially when present on plasmids capable of replicating in prokaryotic host cells such as bacteria.

A host cell according to the present invention preferably is a eukaryotic cell, more preferably a mammalian cell, such as a rodent (e.g. mouse, hamster) cell or a human cell or fusion between different cells. In certain non-limiting embodiments, said host cell is a U-2 OS osteosarcoma, HEK 293, HuNS-1 myeloma, WERI-Rb-1 retinoblastoma, BHK, COS, Vero, non-secreting mouse myeloma Sp2/0-Ag 14, non-secreting mouse myeloma NS0, NCI-H295R adrenal gland carcinomal or a PER.C6^{®} cell. PER.C6 cells for the purpose of the present invention means cells from an upstream or downstream passage or a descendent of an upstream or downstream passage of cells as deposited under ECACC no. 96022940 (see e.g. US patent 5,994,128), i.e. having the characteristics of those cells. It has been previously shown that such cells are capable of expression of proteins at high levels (e.g. WO 00/63403, and Jones et al, 2003). In certain preferred embodiments, the host cells are CHO (Chinese hamster ovary) cells, for instance CHO-K1, CHO-S, CHO-DG44, CHO-DUKXB11, and the like. In certain embodiments, said CHO cells have a dhfr⁻ phenotype.

Such eukaryotic host cells can express desired polypeptides, and are often used for that purpose. They can be obtained by introduction of a DNA molecule of the invention, preferably in the form of an expression cassette, into the cells. Preferably, the expression cassette is integrated in the genome of the host cells, which can be in different positions in various host cells, and selection will provide for a clone where the transgene is integrated in a suitable position, leading to a host cell clone with desired properties in terms of expression levels, stability, growth characteristics, and the like. Alternatively the transcription unit may be targeted or randomly selected for integration into a chromosomal region that is transcriptionally active, e.g. behind a promoter present in the genome.

Preferably the host cells are from a stable clone that can be selected and propagated according to standard procedures known to the person skilled in the art. A culture of such a clone is capable of producing polypeptide of interest, if the cells comprise the transcription unit encoding such.

The invention also provides a method of generating a host cell able to express a polypeptide of interest, said method comprising the steps of: a) introducing into a plurality of precursor cells a DNA molecule or an expression cassette according to the invention, b) culturing the plurality of precursor cells under conditions suitable for expression of the selectable marker polypeptide, and c) selecting at least one host cell expressing the selectable marker polypeptide. Selection for expression of the selectable marker polypeptide is done e.g. by applying selection pressure (e.g. culturing in the presence of selection agent) and will ensure expression of the polypeptide of interest in the multicistronic transcription units and expression cassettes of the invention. This novel method provides a very good result in terms of the ratio of obtained clones versus clones with high expression of the desired polypeptide: far fewer colonies are obtained using the same concentration of selection agent than with known selection systems, and a relatively high percentage of the obtained clones produce the polypeptide of interest at high levels.

The invention further provides a method for producing a polypeptide of interest, comprising culturing a host cell comprising an expression cassette according to the invention, to express the nucleic acid encoding the protein of interest in said cell. In preferred embodiments, the protein of interest is harvested from said cell or from the culture medium or from both. In preferred embodiment, said cell is a mammalian cell, for instance a CHO cell.

Introduction of nucleic acid that is to be expressed in a cell, can be done by one of several methods, which as such are known to the person skilled in the art, also dependent on the format of the nucleic acid to be introduced, Said methods include but are not limited to transfection, infection, injection, transformation, and the like.

In certain embodiments, selection agent is present in the culture medium at least part of the time during the culturing, either in sufficient concentrations to select for cells exposing the selectable marker polypeptide or in lower concentrations. In other embodiments, selection agent is no longer present in the culture medium during the production phase when the polypeptide is expressed.

Culturing a cell is done to enable it to metabolize, and/or grow and/or divide and/or produce recombinant proteins of interest. This can be accomplished by methods well known to persons skilled in the art, and includes but is not limited to providing nutrients for the cell. The methods comprise growth adhering to surfaces, growth in suspension, or combinations thereof. Culturing can be done for instance in dishes, roller bottles or in bioreactors, using batch, fed-batch, continuous systems such as perfusion systems, and the like. In order to achieve large scale (continuous) production of recombinant proteins through cell culture it is preferred in the art to have cells capable of growing in suspension, and it is preferred to have cells capable of being cultured in serum-free, or even protein-free, culture medium.

The conditions for growing or multiplying cells (see e.g. Tissue Culture, Academic Press, Kruse and Paterson, editors (1973)) and the conditions for expression of the recombinant product are known to the person skilled in the art. In general, principles, protocols, and practical techniques for maximizing the productivity of mammalian cell cultures can be found in Mammalian Cell Biotechnology: a Practical Approach (M. Butler, ed., IRL Press, 1991).

In a preferred embodiment, the expressed protein is collected (isolated), either from the cells or from the culture medium or from both. It may then be further purified using known methods, e.g. filtration, column chromatography, etc, by methods generally known to the person skilled in the art.

Obviously, the configurations of the expression cassettes may also be used when the ultimate goal is not the production of a polypeptide of interest, but the RNA itself, for instance for producing increased quantities of RNA from an expression cassette, which may be used for purposes of regulating other genes (e.g. RNAi, antisense RNA), gene therapy, in vitro protein production, etc.

The practice of this invention will employ, unless otherwise indicated, conventional techniques of immunology, molecular biology, microbiology, cell biology, and recombinant DNA, which are within the skill of the art. See e.g. Sambrook, Fritsch and Maniatis, Molecular Cloning: A Laboratory Manual, 2nd edition, 1989; Current Protocols in Molecular Biology, Ausubel FM, et al, eds, 1987; the series Methods in Enzymology (Academic Press, Inc.); PCR2: A Practical Approach, MacPherson MJ, Hams BD, Taylor GR, eds, 1995; Antibodies: A Laboratory Manual, Harlow and Lane, eds, 1988.

The invention is further explained in the following examples. The examples do not limit the invention in any way. They merely serve to clarify the invention.

### EXAMPLES

### Example 1: Removing CpG dinucleotides from the selectable marker coding sequence improves expression using a selection method of the invention

Selection methods using different translation initiation codons for the selectable marker, such as GTG or TTG, can result in very stringent selection, and in very high levels of production for the polypeptide of interest (see WO 2006/048459 and US 2006/0141577, e.g. examples 1-19 in the latter). In this example, the coding region of the selectable marker polypeptide gene itself was modified by removing CpG dinucleotides. The rationale is that the C nucleotide in the CpG nucleotide may be prone to methylation, which might result in gene silencing of the selectable marker, and thus removing CpG dinucleotides might improve the results. The zeocin resistance gene with a TTG startcodon was taken as the marker, and as many CpG dinucleotides were removed as was possible, without changing.the amino acid sequence of the zeocin resistance protein, and further without introducing ATG sequences in the coding strand, to prevent undesired translation initiation within the coding region of the zeocin resistance proteins (as explained e.g in WO 2006/048459). Hence, some CpG's were not removed. The CpG content of the native sequence (here: containing a TTG. startcodon, and a mutations to remove the internal ATG sequence) is 13.3%, whereas after mutating the CpG.'s, the CpG content was reduced to 1.8% [referred to as `TTG Zeo (CpG poor)']. The zeocin resistance gene with decreased CpG content was cloned upstream of the d2EGFP coding sequence to result in a multicistronic expression construct. Expression levels of d2EGFP were measured.

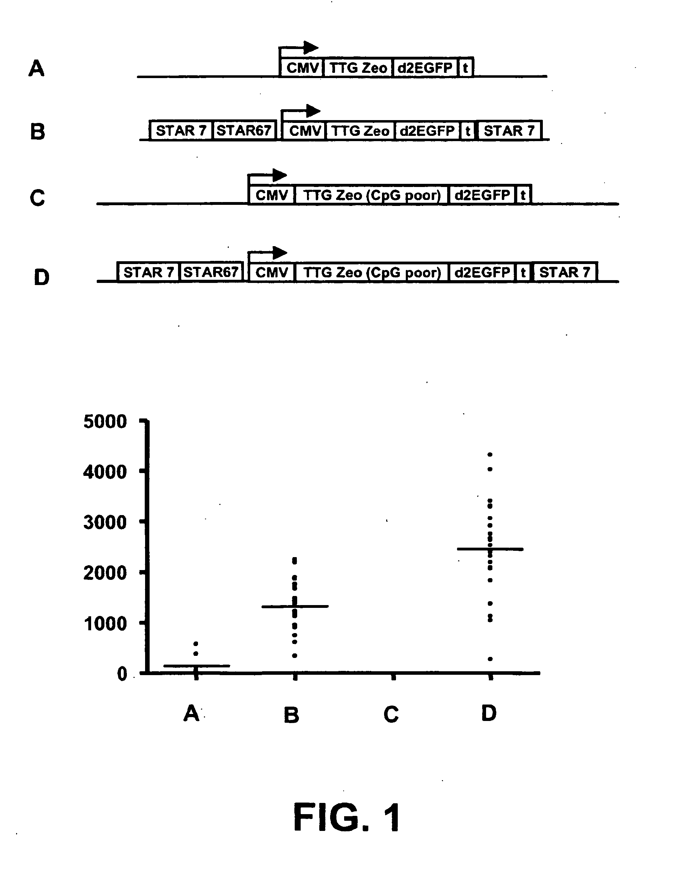

Constructs were prepared containing STARs 7 and 67 upstream of the CMV promoter, followed by the TTG Zeo (CpG poor) selection marker (synthesized by GeneArt GmbH, Regensburg, Germany; see SEQ. ID. NO 3; see SEQ. ID. NO. 1 for the zeocin resistance coding sequence with its natural CpG content), the d2EGFP gene and STAR 7 (Fig. 1). The constructs were transfected to CHO-K1 cells. DNA was transfected using Lipofectamine 2000 (Invitrogen) and cells were grown in the presence of 150 µg/ml Zeocin in HAM-F 12 medium (Invitrogen) + 10% FBS (Invitrogen).

Weight colonies emerged after transaction with the control `Cpt3-rich' TTG Zeo construct (A in Fig. 1) and none with the 'CpG-poor' TTG Zeo containing construct (C in Fig. 1). In contrast, with both 'CpG-rich' TTG Zeo (B in Fig. 1) and "CpG-poor' TTG Zeo (D in Fig. t) selection markers more than 24 colonies emerged when STARs 7/67-7 was included in the construct. With the 'CpG-rich' TTC Zeocin selection market (A in Fig. 1), the average d2EGFP expression with the STAR-less control construct was 140, and with the STAR containing construct 1332 (B in Fig. 1). This is an increase due to the presence of the STAR elements. The average d2EGFP expression with the STAR containing construct and the 'CpG-poor' Zeo was 2453 (D in Fig. 1), an almost two-fold increase in comparison with the 'CpG-rich' TTG Zeo (B in Fig. 1). Furthermore, the highest d2EGFP value achieved with the 'CpG-rich' TTG Zeo construct (B) was 2481 and with the 'CpG-poor' TTG Zeo (D) 4308.

We conclude that lowering the CpG content of the Zeocin marker gene raises the stringency of the selection system. This results in higher d2EGFP expression values when STAR elements are included in the construct and no colonies with the control construct.

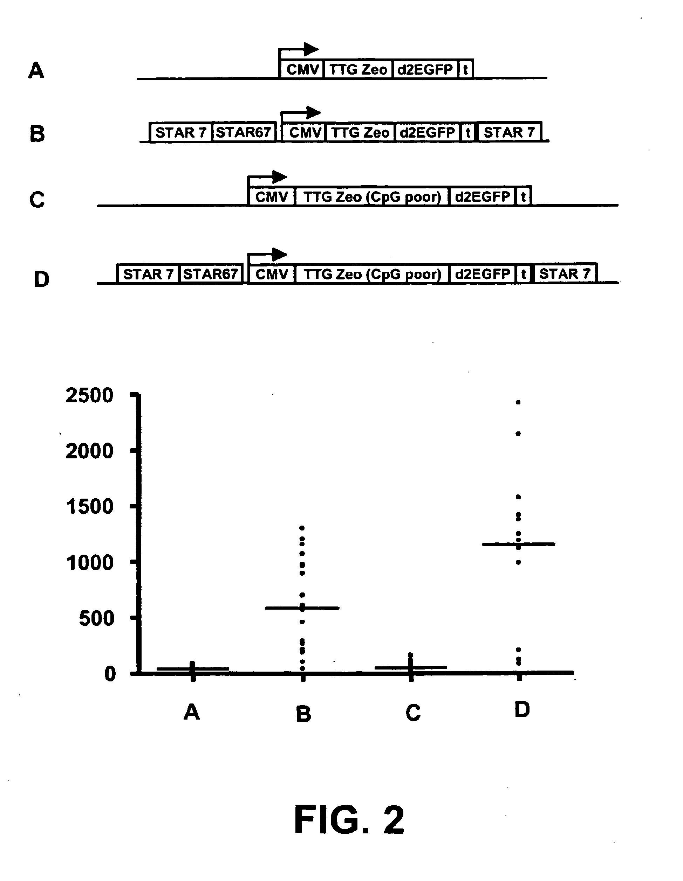

The same constructs were also transfected to CHO-DG44 cells. This was done with Lipofectamine 2000 (Invitrogen) and selection was performed with 150 µg/ml Zeocin in the culture medium. The culture medium consisted of HAMF12:DMEM = 1:1, + 10% foetal bovine serum. With the 'CpG-rich' TTG Zeocin selection marker, the average d2EGFP expression with the STAR-less control construct was 43 (A in Fig. 2), and the average d2EGFP expression with the STAR containing constructs was 586 (B in Fig. 2). This is an increase due to the presence of the STAR elements. The average d2EGFP expression with the STAR constructs and the 'CpG-poor' Zeo was 1152 (D in Fig. 2), an almost two-fold increase in comparison with the 'CpG-rich' TTG Zeo (B in Fig. 2). Furthermore, the highest d2EGFP value achieved with the 'CpG-rich' TTG Zeo construct was 1296 (B in Fig. 2) and with the 'CpG-poor' TTG Zeo 2416 (D in Fig. 2). In contrast with CHO-K1, where no control colonies emerged with the 'CpG-poor' TTG Zeo construct (C in Fig. 1), control colonies emerged with CHO-DG44, but the average d2EGFP value was 52 and the highest value in a colony was 115 (C in Fig. 2).

We conclude that also in CHO-DG44 addition of the 'CpG-poor' TTG Zeo selection marker to the construct results in higher protein expression when STAR elements are employed.

### Example 2: Modifications in the neomycin resistance coding sequence in the selection system of the invention

In this example, besides the startcodon, also the coding region of the neomycin resistance gene was modified, by removing as many CpG dinucleotides of the (ATG-less, so already devoid of ATG sequences in the coding strand) neomycin resistance gene as possible, while not changing the amino acid sequence of the neomycin resistance protein (except for the Met>Leu mutations where the internal ATG sequences were in-frame and replaced by CTG as compared to the wild-type sequence: obviously this was done for reasons of removing ATG sequences from the coding strand and independent from the effort of reducing the CpG content, see example 17 of WO 2006/048459), and without introducing new ATG sequences in the coding strand, analogously to what was done in example 1 for the zeocin resistance gene. The CpG content of the 'wild type' neomycin selection marker gene is 10.4% (SEQ. ID. NO. 5), while after the changes the CpG content was reduced to 2.3% (SEQ. ID. NO. 9). Constructs containing the sequences for the neomycin resistance gene in this example were ordered from GeneArt GmbH, Regensburg, Germany. As a startcodon, TTG was used in this example. The sequences used therefore consisted of SEQ. ID. NO. 9, with the proviso that the startcodon (first three nucleotides, ATG) was replaced by a TTG startcodon, and further in certain cases contained one of the mutations indicated below.

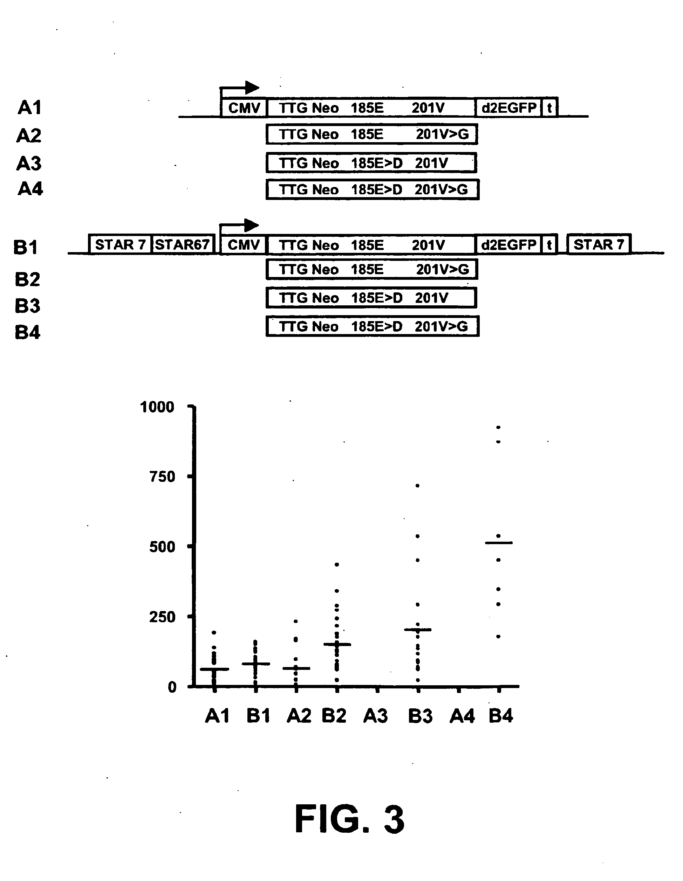

In the 'CpG poor' neomycin resistance gene, some mutations were made to change amino acids in the neomycin resistance protein, to test whether these have influence on the expression levels of the polypeptide of interest when used in the multicistronic transcription units of the invention. The mutations (Sautter et al, 2005; it is noted that the neo sequence used in the present application encodes three additional amino acids immediately after the startcodon as compared to the sequence used by (Sautter et al, 2005), and hence the amino acid numbering in the present application is three higher as compared to the numbering in (Sautter et al, 2005)) consisted of a change from amino acid valine 201 (198 in Sautter et al, 2005) to glycine 201 (TTG Neo 201V>G), glutamic acid 185 (182 in Sautter et al, 2005) to aspartic acid 185 (TTG Neo 185E>D) and a double mutation in which both amino acid valine 201 and glutamic acid 185 were changed to glycine 201 and aspartic acid 185, respectively (TTG Neo 185E>D/ 201 V>G) (Fig. 3). These modifications were compared with the control Neomycin (CpG poor TTG Neo 185E/ 201 V). In all cases constructs were prepared with and without STAR elements (Fig. 3).

The modified TTG Neo selection marker was incorporated in a construct containing STARs 7 and 67 upstream of the CMV promoter, followed by the TTG Neo selection marker, the d2EGFP gene and STAR 7 (Fig. 3). The constructs were transfected to CHO-Kl cells. DNA was transfected using Lipofectamine 2000 (Invitrogen) and cells were grown in the presence of 500 µg/ml G418 geneticin in HAM-F12 medium (Invitrogen) + 10% FBS (Invitrogen).

With the control Neo constmct (185E/201V) only a very limited effect of STAR elements was observed. This may at least in part be due to the numerous colonies that were generated under 500 µg/ml G418 geneticin, indicating that the stringency of the TTG neomycin modification is low. However, the neomycin with modifications of the invention is operational: in the TTG Neo 185E 201 V construct all ATGs were removed from the coding strand of the neomycin resistance gene, and although d2EGFP values were low, it is clear that the removal of ATGs still allowed proper selection under Geneticin selection pressure. When the Neomycin resistance gene was further modified, a distinctive effect of the addition of STAR elements was observed. The mean of 21 TTG Neo 201 V>G control colonies was 65 (A2 in Fig. 3), whereas the mean d2EGFP signal of the 24 TTG Neo 201 V>G colonies with STAR elements was 150 (B2 in Fig. 3). The selection stringency with the TTG Neo 185E>D mutation was further increased, since no control colonies survived without STAR elements (A3 in Fig. 3), whereas the mean d2EGFP signal of 17 surviving TTG Neo 185E>D STAR colonies was 204 (B3 in Fig. 3). This mean GFP fluorescence is higher than with the TTG Neo 201V>G colonies (B2 in Fig. 3). Also the highest d2EGFP value in TTG Neo 185E>D colonies was 715, as compared to 433 in the TTG Neo 201V>G colonies (compare B3 and B2 in Fig. 3). The highest stringency was observed in the double Neo mutant, TTG Neo 185E>D 201 V>G. No control colonies survived (A4 in Fig. 3) and the mean d2EGFP value of 7 surviving STAR TTG Neo 185E>D 201V>G colonies was 513, with as highest d2EGFP value 923 (B4 in Fig. 3).

It is concluded that the introduction of specific mutations raises the stringency of selection of the Neomycin resistance gene when used according to the invention. Some of these modifications convey such selection stringency to the Neomycin resistance gene that only after incorporation with STAR elements colonies are able to survive, due to higher expression values. This concomitantly results in higher d2EGFP expression values. Clearly, the advantageous embodiments described herein of the neomycin resistance gene further improve the suitability of this gene for use according to the present invention.

It will be clear that the configuration where a neomycin resistance gene with decreased CpG content and with a GTG or TTG startcodon, and with the indicated mutations (185E>D and/or 201V>G) could also be placed downstream from the coding sequence for the polypeptide of interest (here d2EGFP as a model) when the neomycin resistance protein coding sequences are placed under control of an IRES (see e.g. example 19 in US 2006/0141577). The same holds for the zeocin resistance gene (example 1). In such case, no care needs to be taken that mutation of CpG dinucleotides would introduce ATG sequences. It is expected that also in such embodiments, good results can be obtained, i.e. that reduction of the CpG content and specific mutation at the indicated positions of the selectable marker protein coding sequence will improve expression levels.

### REFERENCES

Jones D, Kroos N, Anema R, Van Montfort B, Vooys A, Van Der Kraats S, Van Der Helm E, Smits S, Schouten J, Brouwer K, Lagerwerf F, Van Berkel P, Opstelten D-J, Logtenberg T, Bout A (2003) High-level expression of recombinant IgG in the human cell line PER:C6. Biotechnol. Prog. 19: 163-168.
Kozak M. (1986) Point mutations define a sequence flanking the AUG initiator codon that modulates translation by eukaryotic ribosomes. Cell 44: 283-292.
Kozak M. (1987) An analysis of 5'-noncoding sequences from 699 vertebrate messenger RNAs. Nucleic Acids Res. 15: 8125-8148.
Kozak M. (1989) Context effects and inefficient initiation at non-AUG codons in eucaryotic cell-free translation systems. Mol Cell Biol. 9: 5073-5080.
Kozak M. (1990) Downstream secondary structure facilitates recognition of initiator codons by eukaryotic ribosomes. Proc Natl Acad Sci USA 87:8301-8305.
Kozak M. (1997) Recognition of AUG and alternative initiator codons is augmented by G in position +4 but is not generally affected by the nucleotides in positions +5 and +6. EMBO J. 16: 2482-2492.
Kozak M. (2002) Pushing the limits of the scanning mechanism for initiation of translation. Gene 299: 1-34.
Martinez-Salas, E. (1999) Internal ribosome entry site biology and its use in expression vectors Curr Opin Biotechnol 10, 458-64.
Mizuguchi, H, Xu, Z, Ishii-Watabe, A, Uchida, E, and Hayakawa, T. (2000) IRES-dependent second gene expression is significantly lower than cap- dependent first gene expression in a bicistronic vector Mol Ther 1, 376-82.
Rees, S, Coote, J, Stables, J, Goodson, S, Harris, S, and Lee, MG. (1996) Bicistronic vector for the creation of stable mammalian cell lines that predisposes all antibiotic-resistant cells to express recombinant protein Biotechniques 20, 102-104, 106, 108-110.
Sautter, K, Enenkel, B. 2005. Selection of high-producing CHO cells using NPT selection marker with reduced enzyme activity. Biotechnol Bioeng. 89, 530-538.
Venkatesan, A, and Dasgupta, A. (2001) Novel fluorescence-based screen to identify small synthetic internal ribosome entry site elements Mol Cell Biol 21, 2826-37.
Whitelaw, E, Sutherland, H, Kearns, M, Morgan, H, Weaving, L, and Garrick, D. (2001) Epigenetic effects on transgene expression Methods Mol Biol 158, 351-68.

## Claims

1. A DNA molecule comprising an open reading frame sequence that encodes a selectable marker polypeptide that provides resistance against zeocin or against neomycin, ***characterized in that***
said DNA molecule in the coding strand for the selectable marker polypeptide has a GTG startcodon or a TTG startcodon, *and **in that***
the open reading frame sequence that encodes the selectable marker protein has been mutated to replace at least half of its CpG dinucleotides as compared to the native open reading frame sequence that encodes the selectable marker protein.

2. The DNA molecule of claim 1, wherein said startcodon is TTG.

3. The DNA molecule of claim 1 or 2, comprising an open reading frame sequence that encodes a polypeptide that provides resistance against zeocin, wherein the DNA molecule comprises a sequence chosen from the group consisting of:
a) SEQ. ID. NO. 1, with the proviso that at least half of the CpG dinucleotides has been replaced without mutating the amino acid sequence that is encoded, and with the further proviso that the startcodon is either GTG or TTG; and
b) SEQ. ID. NO. 1 wherein nucleotide A at position 280 is replaced by T, and with the proviso that at least half of the CpG dinucleotides has been replaced without mutating the amino acid sequence that is encoded, and with the further proviso that the startcodon is either GTG or TTG.

4. The DNA molecule of claim 3, comprising SEQ. ID. NO. 3.

5. The DNA molecule of claim 1 or 2, comprising an open reading frame sequence that encodes a polypeptide that provides resistance against neomycin, wherein the DNA molecule comprises a sequence chosen from the group consisting of:
a) SEQ. ID. NO. 5, with the proviso that at least half of the CpG dinucleotides has been replaced without mutating the amino acid sequence that is encoded, and with the further proviso that the startcodon is either GTG or TTG; and
b) SEQ. ID. NO. 7, with the proviso that at least half of the CpG dinucleotides of the coding strand has been replaced without mutating the amino acid sequence that is encoded, and with the further proviso that the startcodon is either GTG or TTG; and
c) SEQ. ID. NO. 5 or SEQ. ID. NO. 7, with the proviso that it contains a mutation to encode either of the following polypeptide variants as compared to the polypeptide encoded by the native sequences:
(i) substitution of valine at position 201 into glycine (201V>G), or
(ii) subtitution of glutamic acid at position 185 into aspartic acid (185E>D), or
(iii) a combination of both mutations (i) and (ii) (185E>D and 201V>G),
with the further proviso that at least half of the CpG dinucleotides of the coding strand has been replaced without further mutating the amino acid sequence that is encoded beyond the mutation indicated under (i)-(iii), and with the further proviso that the startcodon is either GTG or TTG.

6. The DNA molecule of claim 5, comprising SEQ. ID. NO. 9, with the proviso that nucleotide A at position 555 is replaced by C, and that nucleotide T at position 602 is replaced by G and that nucleotide G at position 603 is replaced by T, and with the further proviso that the startcodon is either GTG or TTG.

7. The DNA molecule of any one of claims 1-6, wherein the open reading frame sequence that encodes a selectable marker polypeptide is part of a multicistronic transcription unit that further comprises an open reading frame sequence encoding a polypeptide of interest.

8. The DNA molecule of claim 7, wherein the open reading frame that encodes the selectable marker polypeptide is upstream of the open reading frame encoding the polypeptide of interest, and wherein the open reading frame that encodes the selectable marker polypeptide has no ATG sequence in the coding strand.

9. The DNA molecule of claim 7, wherein the open reading frame encoding the polypeptide of interest is upstream of the open reading frame that encodes the selectable marker polypeptide, and wherein the open reading frame that encodes the selectable marker polypeptide is operably linked to an internal ribosome entry site (IRES).

10. An expression cassette comprising the DNA molecule of any one of claims 7-9, said expression cassette comprising a promoter upstream of said multicistronic expression unit and a transcription termination sequence downstream of the multicistronic expression unit.

11. The expression cassette of claim 10, further comprising at least one chromatin control element.

12. A host cell comprising the DNA molecule of any one of claims 1-9 or an expression cassette of any one of claims 10-11.

13. A method of generating a host cell able to express a polypeptide of interest, said method comprising the steps of:
a) introducing into a plurality of precursor cells a DNA molecule according to any one of claims 7-9 or an expression cassette according to any one of claims 10-11,
b) culturing the plurality of precursor cells under conditions suitable for expression of the selectable marker polypeptide, and
c) selecting at least one host cell expressing the polypeptide of interest.

14. A method of expressing a polypeptide of interest, comprising culturing a host cell comprising the expression cassette of any one of claims 10-11, and expressing the polypeptide of interest from the expression cassette.

15. The method according to claims 14, further comprising harvesting the polypeptide of interest.

## Patentansprüche

1. DNA-Molekül, umfassend eine offene Leserastersequenz, die ein auswählbares Marker-Polypeptid codiert, das Resistenz gegen Zeocin oder gegen Neomycin verleiht, **dadurch gekennzeichnet, dass** das DNA-Molekül im codierenden Strang für das auswählbare Marker-Polypeptid ein GTG-Startcodon oder ein TTG-Startcodon enthält und **dadurch**, dass die offene Leserastersequenz, die das auswählbare Marker-Protein codiert, mutiert ist, um mindestens die Hälfte seiner CpG-Dinucleotide zu ersetzten, verglichen mit der nativen offenen Leserastersequenz, die das auswählbare Marker-Protein codiert.

2. DNA-Molekül nach Anspruch 1, wobei das Startcodon TTG ist.

3. DNA-Molekül nach Anspruch 1 oder 2, umfassend eine offene Leserastersequenz, die ein Polypeptid codiert, das Resistenz gegen Zeocin verleiht, wobei das ONA-Molekül eine Sequenz umfasst, die ausgewählt ist aus der Gruppe bestehend aus:
a) SEQ ID NO:1 mit der Maßgabe, dass mindestens die Hälfte der CpG-Dinucleotide ersetzt ist, ohne dass die codierte Aminosäuresequenz mutiert ist und mit der weiteren Maßgabe, dass das Startcodon entweder GTG oder TTG ist; und
b) SEQ ID NO:1, wobei Nucleotid A an Position 280 ersetzt ist durch T, und mit der Maßgabe, dass mindestens die Hälfte der CpG-Dinucleotide ersetzt ist, ohne dass die codierte Aminosäuresequenz mutiert ist, und mit der weiteren Maßgabe, dass das Startcodon entweder GTG oder TTG ist.

4. DNA-Molekül nach Anspruch 3, umfassend SEQ ID NO:3.

5. DNA-Molekül nach Anspruch 1 oder 2, umfassend eine offene Leserastersequenz, die ein Polypeptid codiert, das Resistenz gegen Neomycin verleiht, wobei das DNA-Molekül eine Sequenz umfasst, die ausgewählt ist aus der Gruppe bestehend aus:
a) SEQ ID NO:5, mit der Maßgabe, dass mindestens die Hälfte der CpG-Dinucleotide ersetzt ist, ohne dass die codierte Aminosäuresequenz mutiert ist, und mit der weiteren Maßgabe, dass das Startcodon entweder GTG oder TTG ist; und
b) SEQ ID NO:7, mit der Maßgabe, dass mindestens die Hälfte der CpG-Dinucleotide des codierenden Strangs ersetzt ist, ohne dass die codierte Aminosäuresequenz mutiert ist, und mit der weiteren Maßgabe, dass das Startcodon entweder GTG oder TTG ist; und
c) SEQ. ID NO:5 oder SEQ ID NO:7, mit der Maßgabe, dass sie eine Mutation enthält, um eines der folgenden Polypeptidvarianten zu codieren verglichen mit dem Polypeptid, das durch die nativen Sequenzen codiert wird:
(i) Substitution von Valin in Position 201 zu Glycin (201V>G), oder
(ii) Substitution von Glutaminsäure in Position 185 zu Asparaginsäure (185E>D), oder
(iii) eine Kombination der beiden Mutationen (i) und (ii) (185E>D und 201V>G),
mit der weiteren Maßgabe, dass mindestens die Hälfte der CpG-Dinucleotide des codierenden Strangs ersetzt ist, ohne die codierte Aminosäuresequenz über die unter (i) bis (iii) angegebene Mutation weiter hinaus zu mutieren, und mit der weiteren Maßgabe, dass das Startcodon entweder GTG oder TTG ist.

6. DNA-Molekül nach Anspruch 5, umfassend SEQ ID NO:9 mit der Maßgabe, dass Nucleotid A in Position 555 durch C ersetzt wird und dass Nucleotid T in Position 602 durch G ersetzt wird und dass Nucleotid G in Position 603 durch T ersetzt wird, und mit der weiteren Maßgabe, dass das Startcodon entweder GTG oder TTG ist.

7. DNA-Molekül nach einem der Ansprüche 1 bis 6, wobei die offene Leserastersequenz, die ein auswählbares Marker-Polypeptid codiert, Teil einer multicistronischen Transkriptionseinheit ist, die ferner eine offene Leserastersequenz umfasst, die ein Polypeptid von Interesse codiert.

8. DNA-Molekül nach Anspruch 7, wobei das offene Leseraster, das das auswählbare Marker-Polypeptid codiert, stromaufwärts liegt vom offenen Leseraster, das das Polypeptid von Interesse codiert, und wobei das offene Leseraster, das das auswählbare Marker-Polypeptid codiert, im codierenden Strang keine ATG-Sequenz hat.

9. DNA-Molekül nach Anspruch 7, wobei das das Polypeptid von Interesse codierende offene Leseraster stromaufwärts vom offenen Leseraster liegt, das das auswählbare Marker-Polypeptid codiert, und wobei das offene Leseraster, das das auswählbare Marker-Polypeptid codiert, funktionell verknüpft ist mit einer internen ribosomalen Eintrittsstelle (IRES).

10. Expressionskassette, umfassend das DNA-Molekül von einem der Ansprüche 7 bis 9, wobei die Expressionskassette einen Promotor umfasst, der stromaufwärts der multicistronischen Expressionseinheit liegt, und eine Transkriptionsterminationssequenz, die stromabwärts der multicistronischen Expressionseinheit liegt.

11. Expressionskassette nach Anspruch 10, ferner umfassend mindestens ein Chromatinkontrollelement.

12. Wirtszelle, die das DNA-Molekül nach einem der Ansprüche 1 bis 9 umfasst oder eine Expressionskassette nach einem der Ansprüche 10 bis 11.

13. Verfahren zur Herstellung einer Wirtszelle, die geeignet ist, um ein Polypeptid von Interesse zu exprimieren, wobei das Verfahren die Schritte umfasst:
a) Einbringen eines DNA-Moleküls nach einem der Ansprüche 7 bis 9 oder einer Expressionskassette nach einem der Ansprüche 10 bis 11 in eine Vielzahl von Vorläuferzellen,
b) Züchten der Vielzahl der Vorläuferzellen unter Bedingungen, die geeignet sind zur Expression des auswählbaren Marker-Polypeptids, und
c) Auswählen mindestens einer Wirtszelle, die das Polypeptid von Interesse exprimiert.

14. Verfahren zur Expression eines Polypeptids von Interesse, umfassend das Züchten einer Wirtszelle, die die Expressionskassette nach einem der Ansprüche 10 und 11 umfasst, und das Exprimieren des Polypeptids von Interesse von der Expressionskassette.

15. Verfahren nach Anspruch 14, ferner umfassend das Ernten des Polypeptids von Interesse.

## Revendications

1. Molécule d'ADN comprenant une séquence de cadre de lecture ouvert qui code pour un polypeptide marqueur sélectionnable qui procure une résistance à la zéocine ou à la néomycine, **caractérisée en ce que** :
ladite molécule d'ADN dans le brin codant pour le polypeptide marqueur sélectionnable possède un codon de départ GTG ou un codon de départ TTG, et **en ce que**
la séquence de cadre de lecture ouvert qui code pour la protéine marqueur sélectionnable a subi une mutation destinée à remplacer au moins la moitié de ces dinucléotides CpG par rapport à la séquence native de cadre de lecture ouvert qui code pour la protéine marqueur sélectionnable.

2. Molécule d'ADN selon la revendication 1, dans laquelle ledit codon de départ est TTG.

3. Molécule d'ADN selon la revendication 1 ou 2, comprenant une séquence de cadre de lecture ouvert qui code pour un polypeptide qui procure une résistance à la zéocine, la molécule d'ADN comprenant une séquence choisie parmi le groupe constitué par :
a) la SEQ ID NO: 1, avec cette réserve qu'au moins la moitié des dinucléotides CpG a été remplacée, sans procéder à une mutation de la séquence d'acide aminé qui est encodée, et avec cette réserve supplémentaire que le codon de départ est soit GTG, soit TTG ; et
b) la SEQ ID NO: 1, dans laquelle le nucléotide A à la position 280 est remplacé par T, et avec cette réserve qu'au moins la moitié des dinucléotides CpG a été remplacée, sans procéder à une mutation de la séquence d'acide aminé qui est encodée, et avec cette réserve supplémentaire que le codon de départ est soit GTG, soit TTG.

4. Molécule d'ADN selon la revendication 3, comprenant la SEQ ID NO: 3.

5. Molécule d'ADN selon la revendication 1 ou 2, comprenant une séquence de cadre de lecture ouvert qui code pour un polypeptide qui procure une résistance à la néomycine, la molécule d'ADN comprenant une séquence choisie parmi le groupe constitué par :
a) la SEQ ID NO: 5, avec cette réserve qu'au moins la moitié des dinucléotides CpG a été remplacée, sans procéder à une mutation de la séquence d'acide aminé qui est encodée, et avec cette réserve supplémentaire que le codon de départ est soit GTG, soit TTG ; et
b) la SEQ ID NO: 7, avec cette réserve qu'au moins la moitié des dinucléotides CpG du brin codant a été remplacée, sans procéder à une mutation de la séquence d'acide aminé qui est encodée, et avec cette réserve supplémentaire que le codon de départ est soit GTG, soit TTG ; et
c) la SEQ ID NO: 5 ou la SEQ ID NO: 7, avec cette réserve qu'elle contient une mutation pour le codage de l'un quelconque des variants polypeptidiques suivants, par rapport au polypeptide encodé par les séquences natives :
(i) une substitution de la valine à la position 201 par la glycine (201V>G) ; ou
(ii) une substitution de l'acide glutamique à la position 185 par l'acide aspartique (185E>D) ; ou
(iii) une combinaison des deux mutations (i) et (ii) (185E>D et 201V>G) avec cette réserve supplémentaire qu'au moins la moitié des dinucléotides CpG du brin codant a été remplacée, sans procéder à une mutation supplémentaire de la séquence d'acide aminé qui est encodée, en dehors des mutations indiquées sous (i)-(iii), et avec cette réserve supplémentaire que le codon de départ est soit GTG, soit TTG.

6. Molécule d'ADN selon la revendication 5, comprenant la SEQ ID NO: 9, avec cette réserve que le nucléotide A à la position 555 a été remplacé par C, et que le nucléotide T à la position 602 a été remplacé par G, et que le nucléotide G à la position 603 a été remplacé par T, et avec cette réserve supplémentaire que le codon de départ est soit GTG, soit TTG.

7. Molécule d'ADN selon l'une quelconque des revendications 1 à 6, dans laquelle la séquence de cadre de lecture ouvert qui code pour un polypeptide marqueur sélectionnable fait partie d'une unité de transcription polycistronique qui comprend en outre une séquence de cadre de lecture ouvert codant pour un polypeptide d'intérêt.

8. Molécule d'ADN selon la revendication 7, dans laquelle le cadre de lecture ouvert qui code pour le polypeptide marqueur sélectionnable est situé en amont du cadre de lecture ouvert codant pour le polypeptide d'intérêt, et dans lequel le cadre de lecture ouvert qui code pour le polypeptide marqueur sélectionnable ne possède pas de séquences ATG dans le brin codant.

9. Molécule d'ADN selon la revendication 7, dans laquelle le cadre de lecture ouvert codant pour le polypeptide d'intérêt est situé en amont du cadre de lecture ouvert qui code pour le polypeptide marqueur sélectionnable, et dans lequel le cadre de lecture ouvert qui code pour le polypeptide marqueur sélectionnable est lié de manière opérante à un site d'entrée de ribosome (IRES).

10. Cassette d'expression comprenant la molécule d'ADN selon l'une quelconque des revendications 7 à 9, ladite cassette d'expression comprenant un promoteur en amont de ladite unité d'expression polycistronique et une séquence de terminaison de transcription en aval de l'unité d'expression polycistronique.

11. Cassette d'expression selon la revendication 10, comprenant en outre au moins un élément de contrôle de la chromatine.

12. Cellule hôte comprenant la molécule d'ADN selon l'une quelconque des revendications 1 à 9 ou cassette d'expression selon l'une quelconque des revendications 10-11.

13. Procédé de génération d'une cellule hôte capable d'exprimer un polypeptide d'intérêt, ledit procédé comprenant les étapes :
a) d'introduction, dans plusieurs cellules précurseurs d'une molécule d'ADN selon l'une quelconque des revendications 7 à 9 ou d'une cassette d'expression selon l'une quelconque des revendications 10-11 ;
b) de mise en culture desdites plusieurs cellules précurseurs dans des conditions appropriées pour l'expression du polypeptide marqueur sélectionnable ; et
c) de sélection d'au moins une cellule hôte exprimant le polypeptide d'intérêt.

14. Procédé d'expression d'un polypeptide d'intérêt, comprenant la mise en culture d'une cellule hôte comprenant la cassette d'expression selon l'une quelconque des revendications 10-11, et l'expression du polypeptide d'intérêt à partir de la cassette d'expression.

15. Procédé selon la revendication 14, comprenant en outre la récolte du polypeptide d'intérêt.


### SEQUENCE LISTING

<110> ChromaGenics B.V.
   otte, Arie P.
   Kwaks, Theodorus H.J.
   Sewalt, Richard G.A.B.
   van Blokland, Rik
<120> selection of host cells expressing protein at high levels
<130> 0117 B WO 00 ORD
<150> EP 06113354.2
   <151> 2006-05-02
<150> US 11/416,490
   <151> 2006-05-02
<160> 10
<170> Patent In version 3.3
<210> 1
   <211> 375
   <212> DNA
   <213> Artificial
<220>
   <223> wt zeocin resistance gene
<220>
   <221> CDS
   <222> (1)..(375)
<400> 1
   atg gcc aag ttg acc agt gcc gtt ccg gtg ctc acc gcg cgc gac gtc      48
   Met Ala Lys Leu Thr Ser Ala Val Pro Val Leu Thr Ala Arg Asp Val
   1               5                   10                  15
   gcc gga gcg gtc gag ttc tgg acc gac cgg ctc ggg ttc tcc cgg gac      96
   Ala Gly Ala Val Glu Phe Trp Thr Asp Arg Leu Gly Phe Ser Arg Asp
               20                  25                  30
   ttc gtg gag gac gac ttc gcc ggt gtg gtc cgg gac gac gtg acc ctg      144
   Phe Val Glu Asp Asp Phe Ala Gly Val Val Arg Asp Asp Val Thr Leu
           35                  40                  45
   ttc atc agc gcg gtc cag gac cag gtg gtg ccg gac aac acc ctg gcc      192
   Phe Ile Ser Ala Val Gln Asp Gln Val Val Pro Asp Asn Thr Leu Ala
       50                  55                  60
   tgg gtg tgg gtg cgc ggc ctg gac gag ctg tac gcc gag tgg tcg gag      240
   Trp Val Trp Val Arg Gly Leu Asp Glu Leu Tyr Ala Glu Trp Ser Glu
   65                  70                  75                  80
   gtc gtg tcc acg aac ttc cgg gac gcc tcc ggg ccg gcc atg acc gag      288
   Val Val Ser Thr Asn Phe Arg Asp Ala Ser Gly Pro Ala Met Thr Glu
                   85                  90                  95
   atc ggc gag cag ccg tgg ggg cgg gag ttc gcc ctg cgc gac ccg gcc      336
   Ile Gly Glu Gln Pro Trp Gly Arg Glu Phe Ala Leu Arg Asp Pro Ala
               100                 105                 110
   ggc aac tgc gtg cac ttc gtg gcc gag gag cag gac tga                  375
   Gly Asn Cys Val His Phe Val Ala Glu Glu Gln Asp
           115                 120
<210> 2
   <211> 124
   <212> PRT
   <213> Artificial
<220>
   <223> Synthetic Construct
<400> 2
   Met Ala Lys Leu Thr Ser Ala Val Pro Val Leu Thr Ala Arg Asp Val
   1 5 10 15
   Ala Gly Ala Val Glu Phe Trp Thr Asp Arg Leu Gly Phe Ser Arg Asp
   20 25 30
   Phe Val Glu Asp Asp Phe Ala Gly Val Val Arg Asp Asp Val Thr Leu
   35 40 45
   Phe Ile Ser Ala Val Gln Asp Gln Val Val Pro Asp Asn Thr Leu Ala
   50 55 60
   Trp Val Trp Val Arg Gly Leu Asp Glu Leu Tyr Ala Glu Trp Ser Glu
   65 70 75 80
   Val Val Ser Thr Asn Phe Arg Asp Ala Ser Gly Pro Ala Met Thr Glu
   85 90 95
   Ile Gly Glu Gln Pro Trp Gly Arg Glu Phe Ala Leu Arg Asp Pro Ala
   100 105 110
   Gly Asn Cys Val His Phe Val Ala Glu Glu Gln Asp
   115 120
<210> 3
   <211> 375
   <212> DNA
   <213> Artificial
<220>
   <223> CpG poor and ATG-less zeocin (zeo) resistance sequence
<220>
   <221> CDS
   <222> (1)..(375)
<400> 3
   ttg gcc aag ttg acc agt gct gtc cca gtg ctc aca gcc agg gac gtg 48
   Leu Ala Lys Leu Thr Ser Ala Val Pro Val Leu Thr Ala Arg Asp Val
   1 5 10 15
   gct gga gct gtt gag ttc tgg act gac agg ttg ggg ttc tcc aga gat 96
   Ala Gly Ala Val Glu Phe Trp Thr Asp Arg Leu Gly Phe Ser Arg Asp
   20 25 30
   ttt gtg gag gac gac ttt gca ggt gtg gtc aga gac gac gtc acc ctg 144
   Phe Val Glu Asp Asp Phe Ala Gly Val Val Arg Asp Asp Val Thr Leu
   35 40 45
   ttc atc tca gca gtc cag gac cag gtg gtg cct gac aac acc ctg gct      192
   Phe Ile Ser Ala Val Gln Asp Gln Val Val Pro Asp Asn Thr Leu Ala
       50                      55                      60
   tgg gtg tgg gtg aga gga ctg gac gag ctg tac gct gag tgg agt gag      240
   Trp Val Trp Val Arg Gly Leu Asp Glu Leu Tyr Ala Glu Trp Ser Glu
   65                      70                      75                  80
   gtg gtc tcc acc aac ttc agg gac gcc agt ggc cct gcc ttg aca gag      288
   Val Val Ser Thr Asn Phe Arg Asp Ala Ser Gly Pro Ala Leu Thr Glu
                   85                      90                      95
   att gga gag cag ccc tgg ggg aga gag ttt gcc ctg aga gac cca gca      336
   Ile Gly Glu Gln Pro Trp Gly Arg Glu Phe Ala Leu Arg Asp Pro Ala
               100                     105                     110
   ggc aac tgt gtg cac ttt gtg gca gag gag cag gac tga                  375
   Gly Asn Cys Val His Phe Val Ala Glu Glu Gln Asp
           115                     120
<210> 4
   <211> 124
   <212> PRT
   <213> Artificial
<220>
   <223> synthetic construct
<400> 4
   Leu Ala Lys Leu Thr Ser Ala Val Pro Val Leu Thr Ala Arg Asp Val
   1               5                   10                  15
   Ala Gly Ala Val Glu Phe Trp Thr Asp Arg Leu Gly Phe Ser Arg Asp
               20                  25                  30
   Phe Val Glu Asp Asp Phe Ala Gly Val Val Arg Asp Asp Val Thr Leu
           35                  40                  45
   Phe Ile Ser Ala Val Gln Asp Gln Val Val Pro Asp Asn Thr Leu Ala
       50                  55                  60
   Trp Val Trp Val Arg Gly Leu Asp Glu Leu Tyr Ala Glu Trp Ser Glu
   65                  70                  75                  80
   Val Val Ser Thr Asn Phe Arg Asp Ala Ser Gly Pro Ala Leu Thr Glu
                   85                  90                  95
   Ile Gly Glu Gln Pro Trp Gly Arg Glu Phe Ala Leu Arg Asp Pro Ala
               100                 105                 110
   Gly Asn Cys Val His Phe Val Ala Glu Glu Gln Asp
           115                 120
<210> 5
   <211> 804
   <212> DNA
   <213> Artificial
<220>
   <223> wild type neomycin (Neo) resistance sequence
<220>
   <221> CDS
   <222> (1)..(804)
<400> 5
   atg gga tcg gcc att gaa caa gat gga ttg cac gca ggt tct ccg gcc      48
   Met Gly Ser Ala Ile Glu Gln Asp Gly Leu His Ala Gly Ser Pro Ala
   1               5                   10                  15
   gct tgg gtg gag agg cta ttc ggc tat gac tgg gca caa cag aca atc      96
   Ala Trp Val Glu Arg Leu Phe Gly Tyr Asp Trp Ala Gln Gln Thr Ile
               20                  25                  30
   ggc tgc tct gat gcc gcc gtg ttc cgg ctg tca gcg cag ggg cgc ccg      144
   Gly Cys Ser Asp Ala Ala Val Phe Arg Leu Ser Ala Gln Gly Arg Pro
           35                  40                  45
   gtt ctt ttt gtc aag acc gac ctg tcc ggt gcc ctg aat gaa ctg cag      192
   Val Leu Phe Val Lys Thr Asp Leu Ser Gly Ala Leu Asn Glu Leu Gln
       50                  55                  60
   gac gag gca gcg cgg cta tcg tgg ctg gcc acg acg ggc gtt cct tgc      240
   Asp Glu Ala Ala Arg Leu Ser Trp Leu Ala Thr Thr Gly Val Pro Cys
   65                  70                  75                  80
   gca gct gtg ctc gac gtt gtc act gaa gcg gga agg gac tgg ctg cta      288
   Ala Ala Val Leu Asp Val Val Thr Glu Ala Gly Arg Asp Trp Leu Leu
                   85                  90                  95
   ttg ggc gaa gtg ccg ggg cag gat ctc ctg tca tct cac ctt gct cct      336
   Leu Gly Glu Val Pro Gly Gln Asp Leu Leu Ser Ser His Leu Ala Pro
               100                 105                 110
   gcc gag aaa gta tcc atc atg gct gat gca atg cgg cgg ctg cat acg      384
   Ala Glu Lys Val Ser Ile Met Ala Asp Ala Met Arg Arg Leu His Thr
           115                 120                 125
   ctt gat ccg gct acc tgc cca ttc gac cac caa gcg aaa cat cgc atc      432
   Leu Asp Pro Ala Thr Cys Pro Phe Asp His Gln Ala Lys His Arg Ile
       130                 135                 140
   gag cga gca cgt act cgg atg gaa gcc ggt ctt gtc gat cag gat gat      480
   Glu Arg Ala Arg Thr Arg Met Glu Ala Gly Leu Val Asp Gln Asp Asp
   145                 150                 155                 160
   ctg gac gaa gag cat cag ggg ctc gcg cca gcc gaa ctg ttc gcc agg      528
   Leu Asp Glu Glu His Gln Gly Leu Ala Pro Ala Glu Leu Phe Ala Arg
                   165                 170                 175
   ctc aag gcg cgc atg ccc gac ggc gag gat ctc gtc gtg acc cat ggc      576
   Leu Lys Ala Arg Met Pro Asp Gly Glu Asp Leu Val Val Thr His Gly
               180                 185                 190
   gat gcc tgc ttg ccg aat atc atg gtg gaa aat ggc cgc ttt tct gga      624
   Asp Ala Cys Leu Pro Asn Ile Met Val Glu Asn Gly Arg Phe Ser Gly
           195                 200                 205
   ttc atc gac tgt ggc cgg ctg ggt gtg gcg gac cgc tat cag gac ata      672
   Phe Ile Asp Cys Gly Arg Leu Gly Val Ala Asp Arg Tyr Gln Asp Ile
       210                 215                 220
   gcg ttg gct acc cgt gat att gct gaa gag ctt ggc ggc gaa tgg gct      720
   Ala Leu Ala Thr Arg Asp Ile Ala Glu Glu Leu Gly Gly Glu Trp Ala
   225                 230                 235                 240
   gac cgc ttc ctc gtg ctt tac ggt atc gcc gct ccc gat tcg cag cgc      768
   Asp Arg Phe Leu Val Leu Tyr Gly Ile Ala Ala Pro Asp Ser Gln Arg
                   245                 250                 255
   atc gcc ttc tat cgc ctt ctt gac gag ttc ttc tga                      804
   Ile Ala Phe Tyr Arg Leu Leu Asp Glu Phe Phe
               260                 265
<210> 6
   <211> 267
   <212> PRT
   <213> Artificial
<220>
   <223> synthetic Construct
<400> 6
   Met Gly Ser Ala Ile Glu Gln Asp Gly Leu His Ala Gly Ser Pro Ala
   1 5 10 15
   Ala Trp Val Glu Arg Leu Phe Gly Tyr Asp Trp Ala Gln Gln Thr Ile
   20 25 30
   Gly Cys Ser Asp Ala Ala Val Phe Arg Leu Ser Ala Gln Gly Arg Pro
   35 40 45
   Val Leu Phe Val Lys Thr Asp Leu Ser Gly Ala Leu Asn Glu Leu Gln
   50 55 60
   Asp Glu Ala Ala Arg Leu Ser Trp Leu Ala Thr Thr Gly Val Pro Cys
   65 70 75 80
   Ala Ala Val Leu Asp Val Val Thr Glu Ala Gly Arg Asp Trp Leu Leu
   85 90 95
   Leu Gly Glu Val Pro Gly Gln Asp Leu Leu Ser Ser His Leu Ala Pro
   100 105 110
   Ala Glu Lys Val Ser Ile Met Ala Asp Ala Met Arg Arg Leu His Thr
   115 120 125
   Leu Asp Pro Ala Thr Cys Pro Phe Asp His Gln Ala Lys His Arg Ile
   130 135 140
   Glu Arg Ala Arg Thr Arg Met Glu Ala Gly Leu Val Asp Gln Asp Asp
   145 150 155 160
   Leu Asp Glu Glu His Gln Gly Leu Ala Pro Ala Glu Leu Phe Ala Arg
   165 170 175
   Leu Lys Ala Arg Met Pro Asp Gly Glu Asp Leu Val Val Thr His Gly
   180 185 190
   Asp Ala Cys Leu Pro Asn Ile Met Val Glu Asn Gly Arg Phe Ser Gly
   195 200 205
   Phe Ile Asp Cys Gly Arg Leu Gly Val Ala Asp Arg Tyr Gln Asp Ile
   210 215 220
   Ala Leu Ala Thr Arg Asp Ile Ala Glu Glu Leu Gly Gly Glu Trp Ala
   225 230 235 240
   Asp Arg Phe Leu Val Leu Tyr Gly Ile Ala Ala Pro Asp Ser Gln Arg
   245 250 255
   Ile Ala Phe Tyr Arg Leu Leu Asp Glu Phe Phe
   260 265
<210> 7
   <211> 804
   <212> DNA
   <213> Artificial
<220>
   <223> modified neomycin resistance gene lacking internal ATG sequences
<220>
   <221> CDS
   <222> (1)..(804)
<400> 7
   atg gga tcg gcc att gaa caa gac gga ttg cac gca ggt tct ccg gcc      48
   Met Gly Ser Ala Ile Glu Gln Asp Gly Leu His Ala Gly Ser Pro Ala
   1               5                   10                  15
   gct tgg gtg gag agg cta ttc ggc tac gac tgg gca caa cag aca atc      96
   Ala Trp Val Glu Arg Leu Phe Gly Tyr Asp Trp Ala Gln Gln Thr Ile
               20                  25                  30
   ggc tgc tct gac gcc gcc gtg ttc cgg ctg tca gcg cag ggg cgc ccg      144
   Gly Cys Ser Asp Ala Ala Val Phe Arg Leu Ser Ala Gln Gly Arg Pro
           35                  40                  45
   gtt ctt ttt gtc aag acc gac ctg tcc ggt gcc ctg aac gaa ctg cag      192
   Val Leu Phe Val Lys Thr Asp Leu Ser Gly Ala Leu Asn Glu Leu Gln
       50                  55                  60
   gac gag gca gcg cgg cta tcg tgg ctg gcc acg acg ggc gtt cct tgc      240
   Asp Glu Ala Ala Arg Leu Ser Trp Leu Ala Thr Thr Gly Val Pro Cys
   65                  70                  75                  80
   gca gct gtg ctc gac gtt gtc act gaa gcg gga agg gac tgg ctg cta      288
   Ala Ala Val Leu Asp Val Val Thr Glu Ala Gly Arg Asp Trp Leu Leu
                   85                  90                  95
   ttg ggc gaa gtg ccg ggg cag gat ctc ctg tca tct cac ctt gct cct      336
   Leu Gly Glu Val Pro Gly Gln Asp Leu Leu Ser Ser His Leu Ala Pro
               100                 105                 110
   gcc gag aaa gta tcc atc ctg gct gac gca ctg cgg cgg ctg cat acg      384
   Ala Glu Lys Val Ser Ile Leu Ala Asp Ala Leu Arg Arg Leu His Thr
           115                 120                 125
   ctt gat ccg gct acc tgc cca ttc gac cac caa gcg aaa cat cgc atc      432
   Leu Asp Pro Ala Thr Cys Pro Phe Asp His Gln Ala Lys His Arg Ile
       130                 135                 140
   gag cga gca cgt act cgg ctg gaa gcc ggt ctt gtc gat cag gac gat      480
   Glu Arg Ala Arg Thr Arg Leu Glu Ala Gly Leu Val Asp Gln Asp Asp
   145                 150                 155                 160
   ctg gac gaa gag cat cag ggg ctc gcg cca gcc gaa ctg ttc gcc agg      528
   Leu Asp Glu Glu His Gln Gly Leu Ala Pro Ala Glu Leu Phe Ala Arg
                   165                 170                 175
   ctc aag gcg cgc ctg ccc gac ggc gac gat ctc gtc gtg acc cac ggc      576
   Leu Lys Ala Arg Leu Pro Asp Gly Asp Asp Leu Val Val Thr His Gly
               180                 185                 190
   gac gcc tgc ttg ccg aat atc ctg gtg gaa aac ggc cgc ttt tct gga      624
   Asp Ala Cys Leu Pro Asn Ile Leu Val Glu Asn Gly Arg Phe Ser Gly
           195                 200                 205
   ttc atc gac tgt ggc cgg ctg ggt gtg gcg gac cgc tat cag gac ata      672
   Phe Ile Asp Cys Gly Arg Leu Gly Val Ala Asp Arg Tyr Gln Asp Ile
       210                 215                 220
   gcg ttg gct acc cgt gat att gct gaa gag ctt ggc ggc gag tgg gct      720
   Ala Leu Ala Thr Arg Asp Ile Ala Glu Glu Leu Gly Gly Glu Trp Ala
   225                 230                 235                 240
   gac cgc ttc ctc gtg ctt tac ggt atc gcc gct ccc gat tcg cag cgc      768
   Asp Arg Phe Leu Val Leu Tyr Gly Ile Ala Ala Pro Asp Ser Gln Arg
                   245                 250                 255
   atc gcc ttc tat cgc ctt ctt gac gag ttc ttc tga                      804
   Ile Ala Phe Tyr Arg Leu Leu Asp Glu Phe Phe
               260                 265
<210> 8
   <211> 267
   <212> PRT
   <213> Artificial
<220>
   <223> synthetic Construct
<400> 8
   Met Gly Ser Ala Ile Glu Gln Asp Gly Leu His Ala Gly Ser Pro Ala
   1               5                   10                  15
   Ala Trp Val Glu Arg Leu Phe Gly Tyr Asp Trp Ala Gln Gln Thr Ile
               20                  25                  30
   Gly Cys Ser Asp Ala Ala Val Phe Arg Leu Ser Ala Gln Gly Arg Pro
           35                  40                  45
   Val Leu Phe Val Lys Thr Asp Leu Ser Gly Ala Leu Asn Glu Leu Gln
       50                  55                  60
   Asp Glu Ala Ala Arg Leu Ser Trp Leu Ala Thr Thr Gly Val Pro Cys
   65                  70                  75                  80
   Ala Ala Val Leu Asp Val Val Thr Glu Ala Gly Arg Asp Trp Leu Leu
                   85                  90                  95
   Leu Gly Glu Val Pro Gly Gln Asp Leu Leu Ser Ser His Leu Ala Pro
   100 105 110
   Ala Glu Lys Val Ser Ile Leu Ala Asp Ala Leu Arg Arg Leu His Thr
   115 120 125
   Leu Asp Pro Ala Thr Cys Pro Phe Asp His Gln Ala Lys His Arg Ile
   130 135 140
   Glu Arg Ala Arg Thr Arg Leu Glu Ala Gly Leu Val Asp Gln Asp Asp
   145 150 155 160
   Leu Asp Glu Glu His Gln Gly Leu Ala Pro Ala Glu Leu Phe Ala Arg
   165 170 175
   Leu Lys Ala Arg Leu Pro Asp Gly Asp Asp Leu Val Val Thr His Gly
   180 185 190
   Asp Ala Cys Leu Pro Asn Ile Leu Val Glu Asn Gly Arg Phe Ser Gly
   195 200 205
   Phe Ile Asp Cys Gly Arg Leu Gly Val Ala Asp Arg Tyr Gln Asp Ile
   210 215 220
   Ala Leu Ala Thr Arg Asp Ile Ala Glu Glu Leu Gly Gly Glu Trp Ala
   225 230 235 240
   Asp Arg Phe Leu Val Leu Tyr Gly Ile Ala Ala Pro Asp Ser Gln Arg
   245 250 255
   Ile Ala Phe Tyr Arg Leu Leu Asp Glu Phe Phe
   260 265
<210> 9
   <211> 804
   <212> DNA
   <213> Artificial
<220>
   <223> CpG poor Neo resistance sequence
<220>
   <221> CDS
   <222> (1)..(804)
<400> 9
   atg gga agt gcc att gaa caa gac gga ttg cac gca ggt tct cct gca 48
   Met Gly Ser Ala Ile Glu Gln Asp Gly Leu His Ala Gly Ser Pro Ala
   1 5 10 15
   gct tgg gtg gag agg cta ttt ggc tac gac tgg gca caa cag aca ata 96
   Ala Trp Val Glu Arg Leu Phe Gly Tyr Asp Trp Ala Gln Gln Thr Ile
   20 25 30
   ggc tgc tct gac gca gca gtg ttc aga ctg tca gca cag ggg aga cca       144
   Gly Cys Ser Asp Ala Ala Val Phe Arg Leu Ser Ala Gln Gly Arg Pro
           35                  40                  45
   gtt ctt ttt gtc aag act gac ctg tca ggt gcc ctg aac gaa ctg cag       192
   Val Leu Phe Val Lys Thr Asp Leu Ser Gly Ala Leu Asn Glu Leu Gln
       50                  55                  60
   gac gag gca gca aga cta agt tgg ctg gcc act act ggt gtt cct tgt       240
   Asp Glu Ala Ala Arg Leu Ser Trp Leu Ala Thr Thr Gly Val Pro Cys
   65                  70                  75                  80
   gca gct gtg ttg gac gtt gtc act gaa gca gga agg gac tgg ctg cta       288
   Ala Ala Val Leu Asp Val Val Thr Glu Ala Gly Arg Asp Trp Leu Leu
                   85                  90                  95
   ttg ggt gaa gtg cct ggg cag gat ctc ctg tca tct cac ctt gct cct       336
   Leu Gly Glu Val Pro Gly Gln Asp Leu Leu Ser Ser His Leu Ala Pro
               100                 105                 110
   gca gag aaa gta tcc atc ctg gct gac gca ctg aga aga ctg cat act       384
   Ala Glu Lys Val Ser Ile Leu Ala Asp Ala Leu Arg Arg Leu His Thr
           115                 120                 125
   ctt gat cca gct acc tgc cca ttt gac cac caa gca aaa cat aga att       432
   Leu Asp Pro Ala Thr Cys Pro Phe Asp His Gln Ala Lys His Arg Ile
       130                 135                 140
   gag aga gca cga act aga ctg gaa gca ggt ctt gta gat cag gac gat       480
   Glu Arg Ala Arg Thr Arg Leu Glu Ala Gly Leu Val Asp Gln Asp Asp
   145                 150                 155                 160
   ctg gac gaa gag cat cag ggg ttg gca cca gca gaa ctg ttt gcc agg       528
   Leu Asp Glu Glu His Gln Gly Leu Ala Pro Ala Glu Leu Phe Ala Arg
                   165                 170                 175
   ctc aag gca aga ctg cct gac ggt gaa gat ttg gtt gtg acc cac ggt       576
   Leu Lys Ala Arg Leu Pro Asp Gly Glu Asp Leu Val Val Thr His Gly
               180                 185                 190
   gac gcc tgc ttg cct aat atc ctg gtg gaa aac ggc aga ttt tct gga       624
   Asp Ala Cys Leu Pro Asn Ile Leu Val Glu Asn Gly Arg Phe Ser Gly
           195                 200                 205
   ttc att gac tgt ggc aga ctg ggt gtg gca gac aga tat cag gac ata       672
   Phe Ile Asp Cys Gly Arg Leu Gly Val Ala Asp Arg Tyr Gln Asp Ile
       210                 215                 220
   gca ttg gct acc aga gat att gct gaa gag ctt ggt ggt gag tgg gct       720
   Ala Leu Ala Thr Arg Asp Ile Ala Glu Glu Leu Gly Gly Glu Trp Ala
   225                 230                 235                 240
   gac aga ttc ttg gtg ctt tac ggt ata gcc gct cct gat tca cag aga       768
   Asp Arg Phe Leu Val Leu Tyr Gly Ile Ala Ala Pro Asp Ser Gln Arg
                   245                 250                 255
   ata gcc ttc tat aga ctt ctt gac gag ttc ttc tga                       804
   Ile Ala Phe Tyr Arg Leu Leu Asp Glu Phe Phe
               260                 265
<210> 10
   <211> 267
   <212> PRT
   <213> Artificial
<220>
   <223> synthetic Construct
<400> 10
   Met Gly Ser Ala Ile Glu Gln Asp Gly Leu His Ala Gly Ser Pro Ala
   1               5                   10                  15
   Ala Trp Val Glu Arg Leu Phe Gly Tyr Asp Trp Ala Gln Gln Thr Ile
               20                  25                  30
   Gly Cys Ser Asp Ala Ala Val Phe Arg Leu Ser Ala Gln Gly Arg Pro
           35                  40                  45
   Val Leu Phe Val Lys Thr Asp Leu Ser Gly Ala Leu Asn Glu Leu Gln
       50                  55                  60
   Asp Glu Ala Ala Arg Leu Ser Trp Leu Ala Thr Thr Gly Val Pro Cys
   65                  70                  75                  80
   Ala Ala Val Leu Asp Val Val Thr Glu Ala Gly Arg Asp Trp Leu Leu
                   85                  90                  95
   Leu Gly Glu Val Pro Gly Gln Asp Leu Leu Ser Ser His Leu Ala Pro
               100                 105                 110
   Ala Glu Lys Val Ser Ile Leu Ala Asp Ala Leu Arg Arg Leu His Thr
           115                 120                 125
   Leu Asp Pro Ala Thr Cys Pro Phe Asp His Gln Ala Lys His Arg Ile
       130                 135                 140
   Glu Arg Ala Arg Thr Arg Leu Glu Ala Gly Leu Val Asp Gln Asp Asp
   145                 150                 155                 160
   Leu Asp Glu Glu His Gln Gly Leu Ala Pro Ala Glu Leu Phe Ala Arg
                   165                 170                 175
   Leu Lys Ala Arg Leu Pro Asp Gly Glu Asp Leu Val Val Thr His Gly
               180                 185                 190
   Asp Ala Cys Leu Pro Asn Ile Leu Val Glu Asn Gly Arg Phe Ser Gly
           195                 200                 205
   Phe Ile Asp Cys Gly Arg Leu Gly Val Ala Asp Arg Tyr Gln Asp Ile
       210                 215                 220
   Ala Leu Ala Thr Arg Asp Ile Ala Glu Glu Leu Gly Gly Glu Trp Ala
   225                 230                 235                 240
   Asp Arg Phe Leu Val Leu Tyr Gly Ile Ala Ala Pro Asp Ser Gln Arg
                   245                 250                 255
   Ile Ala Phe Tyr Arg Leu Leu Asp Glu Phe Phe
   260 265